# EUROPEAN PATENT APPLICATION

(11) **EP 1 552 857 A1**
(43) Date of publication of application: **13.07.2005**
(21) Application number: 05250098.0
(22) Date of filing: 11.01.2005
(51) Int. Cl.: A61M 11/00, A61M 11/06, A61M 15/00

(54) **Apparatus for dispensing medicaments**

(30) Priority: 12.01.2004 US 756449
(71) Applicant: Hewlett-Packard Development Company, L.P., Houston, TX 77070 (US)
(72) Inventor: Dunfield, Steve, Corvallis, Oregon 97330 (US); Ayres, James, Corvallis, Oregon 97330 (US)
(74) Representative: Jackson, Richard Eric

(57) **Abstract**

A method of dispensing a medicament (24). A treatment plan (84) having at least two rates of action (204) for a medicament (24) may be provided. A droplet characteristic corresponding to each rate of action (204) may be selected. Medicament droplets having each droplet characteristic may be ejected into a mucosal tract (171, 172) according to the treatment plan (84), thereby allowing the medicament (24) to act at two or more rates.

## Description

### BACKGROUND

Medicaments, such as drugs, may be delivered to target tissue within a body by various routes. Each of these routes passes the medicaments through a body surface layer that topologically separates the target tissue from the environment. Accordingly, medicaments may be delivered to target tissue through an external region of the body's surface layer, the skin. Passage through the skin may be achieved by mechanical penetration of the skin, such as by injection, or by topical application and subsequent absorption through the skin, such as with a patch. Alternatively, medicaments may be delivered to target tissue by passage through an internal region of the body's surface layer, that is, through mucous membranes (or mucosae) defined by the respiratory and gastrointestinal systems of the body. For example, medicaments may be ingested (such as from a tablet, a capsule, a lozenge, etc.) for absorption through the mucosae of the gastrointestinal system, or inhaled (such as from a metered-dose inhaler) for absorption through the mucosae of the respiratory system.

A particular medicament may have very different rates of action in the body based on how the medicament is dispensed. For example, the medicament may be injected intravenously to achieve faster action of the medicament, or ingested to achieve slower action of the medicament. Therefore, a delivery route, and thus a mechanism of dispensing each medicament, may be selected according to action kinetics suitable for the medicament. However, a single delivery route may be inadequate to achieve the desired action kinetics of a medicament, such as when the medicament is used to treat different aspects of substance addiction. For example, a cigarette smokers addiction to nicotine may create symptoms of physical withdrawal and a craving for cigarettes (and thus nicotine), if the smoker stops smoking cigarettes. The symptoms and the craving may need to be treated separately using nicotine delivered with a combination of action kinetics as a replacement for smoking.

Physical withdrawal from nicotine may involve various symptoms, such as insomnia, change in appetite, irritability, and depression. Physical withdrawal may be treated with a sustained level of nicotine in the blood of the smoker. Accordingly, a slower rate of nicotine delivery to the blood (a slower rate of action) may be effective for reducing symptoms of physical withdrawal from nicotine. However, this slower rate of action may be relatively unsuccessful at treating the craving for cigarettes.

The craving for cigarettes may relate to a craving for the feelings associated with smoking, produced in part by a rapid increase in nicotine levels in the blood. Accordingly, faster action of the nicotine after the nicotine is dispensed may reduce the craving for cigarettes.

A treatment program for nicotine addiction may employ two different types of dispensing devices to achieve different rates of nicotine delivery to the bloodstream of a person attempting to quit smoking. A nicotine patch provides a slower, more sustained delivery of nicotine to the bloodstream, and a nicotine nasal spray provides a more rapid, less sustained delivery of nicotine to the bloodstream. However, the use of different dispensing devices may increase the complexity of the treatment program and may enhance the probability of failure. In addition, the use of different dispensing devices may make it difficult to place treatment under processor control.

### SUMMARY

A method of dispensing a medicament is employed wherein a treatment plan having at least two rates of action for a medicament may be provided. A droplet characteristic corresponding to each rate of action may be selected. Medicament droplets having each droplet characteristic may be ejected into a mucosal tract according to the treatment plan, thereby allowing the medicament to act at two or more rates.

### BRIEF DESCRIPTION OF THE DRAWINGS

Fig. 1 is a side view of a medicament ejector introducing droplets of a medicament into a person's mouth based on a selected rate of action of the medicament, in accordance with an embodiment of the invention.
Fig. 2 is a schematic view of the medicament ejector of Fig. 1, in accordance with an embodiment of the invention.
Fig. 3 is a view of an ejection mechanism that may be included in the medicament ejector of Fig. 1, in accordance with an embodiment of the invention.
Fig. 4 is a view of an ejection mechanism that may be included in the medicament ejector of Fig. 1, in accordance with an embodiment of the invention.
Fig. 5 is a fragmentary sectional view of selected portions of the ejection mechanism of Fig. 4, viewed generally along line 5-5 of Fig. 4, during ejection of medicament droplets having different sizes in accordance with an embodiment of the invention.
Fig. 6 is a view of an ejection mechanism that may be included in the medicament ejector of Fig. 1, in accordance with an embodiment of the invention.
Fig. 7 is a fragmentary sectional view of selected portions of the ejection mechanism of Fig. 6, viewed generally along line 7-7 of Fig. 6, during ejection of droplets having different sizes from each of two medicament reservoirs in accordance with an embodiment of the invention.
Fig. 8 is a schematic view of a relationship between size and deposition site for particles or fluid droplets dispensed into a respiratory system of a person, in accordance with an embodiment of the invention.
Fig. 9 is a schematic view of aspects of a treatment plan that may be included in the medicament ejector of Figs. 1 and 2, to direct programmatic selection of a rate of action, and thus of a droplet size and a droplet composition, according to the time elapsed since the last drug dose, in accordance with an embodiment of the invention.
Fig. 10 is a graph plotting drug dose relative to dose number for the treatment plan of Fig. 9, in accordance with an embodiment of the invention.
Fig. 11 is a flowchart of a method of programmatically dispensing droplets of medicament to a person, to achieve different rates of action of the medicament, in accordance with an embodiment of the invention.

### DETAILED DESCRIPTION

A system, including method and apparatus, is provided for programmatically dispensing a medicament for different action rates in a person. Each different rate of action may be achieved by selecting a droplet characteristic corresponding to the rate of action. The droplet characteristic may be target deposition site, composition, ejection velocity, and/or the like. For example, different rates of action may be achieved by deposition of the medicament at a different mucosal region within a mucosal tract, such as the respiratory system of the person. The mucosal region (or regions) may be determined by the size (or sizes) of medicament droplets ejected by a medicament ejection device. The medicament droplets may be ejected into the mouth or nose of the person from the ejection device, to allow the droplets to travel to a site of deposition, based on the size of the droplets. For example, larger droplets may be deposited on an upper mucosal region (such as the mucosae of the mouth and nose), and smaller droplets may travel farther into the respiratory system of the body, for deposition on a lower mucosal region (such as the mucosae of the bronchi, bronchioles, and alveoli). The medicament may be absorbed at different rates at these mucosal regions, to provide different rates of medicament action. Therefore, the medicament may be dispensed with increased flexibility, to more effectively accommodate complex treatment plans.

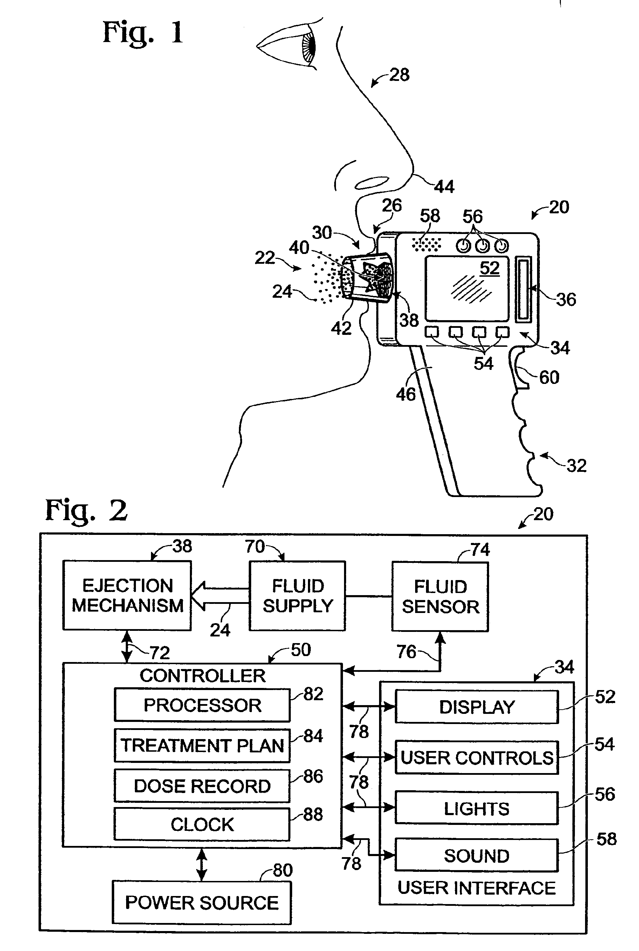

Fig. 1 shows an embodiment of a medicament ejection apparatus or medicament ejector 20. Medicament ejectors may offer processor-controlled delivery of medicaments in fluid. In the present illustration, ejector 20 is ejecting medicament droplets 22 of a medicament 24 into the mouth 26 of a person 28. The medicament droplets 22 may have a size and/or composition based on a desired or selected rate of action of the medicament. Ejector 20 may include an ejection portion 30, a handle portion 32, a user interface 34, and a digital interface 36, among others.

A rate of action, as used herein, includes the rate of onset of an action (or effect) and/or the duration of the action (or effect) of a medicament or drug after it is dispensed. The rate of onset may be related to the rate of input into the body, for example, the rate of absorption of a dispensed or deposited medicament or drug through a mucosal membrane or the skin to reach a target tissue or the bloodstream. The rate of onset also may be related to the concentration of a drug in the medicament and the minimum effective concentration at which the drug produces an action or effect after input. For example, a lower concentration of a drug may have a slower rate of action, because it takes longer to reach the minimum effective concentration of the drug in the bloodstream. Alternatively, or in addition, the rate of action may be influenced by the site of deposition of the medicament (and thus the droplet size), the type(s) and amount(s) of excipient(s) included in the medicament (see below), other droplets ejected proximate to the medicament droplets, and/or the velocity of medicament droplet ejection, among others. The duration of action may be related to the rate of input, for example, a longer duration of action (and slower rate of action) being created by a slower rate of input.

Ejection portion 30 may be any structure configured to create fluid droplets 22 and direct the fluid droplets toward person 28. The ejection portion 30 may include a droplet generator or ejection mechanism 38 to eject the medicament through orifices 40 in the form of medicament droplets 22. The ejection mechanism may include one or a plurality of ejection devices (sets of orifices) and may use aspects of inkjet technology to eject medicament droplets 22, as described further below.

Ejection portion 30 also may include a conduit that directs the medicament droplets toward the person. The conduit may correspond to a mouthpiece 42 configured to be received in or directed towards mouth 26 of the person. Mouthpiece 42 may facilitate inhalation of droplets 22, for example, by enabling the lips of the person to form a temporary seal around the mouthpiece, to direct air flow through the bore of the mouthpiece. Alternatively, the conduit may be configured to be received in one or both nostrils of nose 44, to be received in any other body orifice or mucosal tract, or to be positioned adjacent a person's skin. In some embodiments, the conduit may be bifurcated, branched, or shaped otherwise to facilitate delivery of medicament droplets to two or more body orifices and/or skin regions in parallel and/or sequentially. For example, the conduit may be configured to facilitate droplet delivery selectively into both the mouth and nose.

Handle portion 32 may be any structure configured to enable a person's hand or hands to grip the ejector. Accordingly, handle portion 32 may be defined by a body portion 46 of the ejector, and/or one or more projections extending therefrom. In some embodiments, the ejector may be configured to be supported without the hands of person 28. For example, the ejector may be connected to person 28 with straps, such as a mask or ventilator, or may be positioned by connection to a supporting frame.

User interface 34 may include any input/output devices that provide communication between a person and a controller 50 of ejector 20 (see Fig. 2). The user interface may include a display 52, user controls 54, lights 56, and sound interface 58.

Display 52 may be any output device that produces patterned visual output, such as text, numbers, graphs, or images, based on instructions from controller 50. The display may be used, for example, to provide person 28 with instructions for use, to present aspects of a treatment plan, to present dosage information or usage information, to indicate medicament type or level, to indicate power status, etc.

User controls 54 may be any user-operated device(s) for transducing a user intention into an electrical signal. Exemplary user controls may include buttons, switches, knobs, a keyboard, a keypad, a mouse, etc. In addition, the user controls may include an actuator 60 for signaling ejector 20 that person 28 is ready to receive medicament 24. In the present illustration, actuator 60 is a button to be pressed by person 28. In other embodiments, the actuator may be operated by sound, light, heat, air pressure (such as the pressure produced by exhalation or inhalation), or a biometric aspect of the person. Alternatively, the ejector may be automatic, so that no actuation is necessary from person 28 before the medicament is dispensed. Thus, the ejector may be configured for use by animals other than humans.

Lights 56 may be any light source(s) that emits visible light to person 28. For example, the lights may be LEDs that convey information about the status of ejector 20.

Sound interface 58 may be any input/output device configured to emit sound or sense sound. Accordingly, sound interface 58 may be a speaker that conveys status information to person 28, based on instructions from controller 50. Alternatively, or in addition, sound interface 58 may be a microphone that detects sound, such as a user command, and send signals to controller 50, based on the detected sound.

Digital interface 36 may be any device for exchanging digital information with ejector 20. The digital interface may be a site for creating a communications link with ejector 20, for example, with a separate computing device. Alternatively, the digital interface may be a site for receiving a portable memory device, such as a memory chip, card, or stick, among others. In any case, the digital interface may allow a treatment plan to be loaded into ejector 20, to be modified in situ, or may allow usage data of the ejector to be transferred from the ejector.

Fig. 2 shows a schematic view of medicament ejector 20. Ejector 20 may dispense medicament 24 received from fluid supply 70 using ejection mechanism 38, under the control of controller 50, shown at 72. Aspects of fluid supply 70, such as pressure, level, turbidity, temperature, electrical conductivity, etc., may be sensed by a fluid sensor 74 and communicated to controller 50, shown at 76. User interface 34 may provide inputs to controller 50, and receive outputs from controller 50, shown at 78. Power source 80 may supply power to energize electronic circuitry, which may define aspects of the controller, user interface, ejection mechanism, fluid sensor, and/or connections therebetween.

Controller 50 may provide programmatic selection of medicament compositions), medicament dose(s), medicament rate(s) of action, medicament droplet size(s), and/or time of medicament dispensing, among others. The controller may include a processor 82, a treatment plan 84, a dose record 86, and a clock 88 to perform such selection.

Processor 82 may be any device configured to perform manipulation of data, such as arithmetic and logic operations. The processor may receive input data from user interface 34, such as an actuation request from a user. The processor then may implement the request by sending appropriate energizing signals to ejection mechanism 38 for ejection of medicament, according to instructions in treatment plan 84 and based on dose record 86.

Treatment plan 84 may be any instructions defining aspects of medicament dispensing. Plan 84 may define medicament compositions, medicament doses, medicament rates of action, medicament droplet sizes, and/or medicament temporal ejection schedules, among others. The plan may define a medicament composition, rate of action, droplet size, and/or dose to be administered, based on dose record 86. Each defined medicament composition may be one medicament composition out of two or more available medicament compositions, or may be a mixture of two or more medicament compositions. Each defined medicament dose may be an amount (a volume, a number of drops, a duration of ejection, etc.) of the medicament to be administered. Each defined medicament droplet size may be a size(s) of medicament droplets to be ejected, based on a selected rate(s) of action and thus composition and/or selected deposition site(s) for the medicament. The size may be a diameter of the droplets, for example, as defined by bores (or orifices) from which the medicament is ejected (see below).

Dose record 86 may be any information related to one or more previous doses that were administered or not administered to the user. The information may be acquired with the aid of clock 88 or may be a dosage count that is independent of time. The dose record may include information about each dose (time of dispensing, amount dispensed, medicament composition, action rate, droplet size, etc.), time intervals between doses, number of doses per day, number of administrations to each deposition site per day, missed doses, time since last dose, etc. Clock 88 may be any measuring device that provides current and/or elapsed time.

The medicament, or droplets thereof, may have one or more characteristics selected in correspondence with a desired rate of action of the medicament. The characteristic may be any aspect of the medicament that affects the rate of action after the medicament is dispensed. Accordingly, the characteristic may be droplet size of the medicament, droplet ejection velocity, droplet environment (proximate droplets of other types), and/or droplet composition, such as concentration or type of a bioactive agent in the medicament, concentration or type of excipient in the medicament, and/or the like.

A medicament, as used herein, may be any bioactive composition administered for a therapeutic and/or diagnostic purpose. The term "bioactive composition" (or bioactive agent) as used herein, refers to a composition (or agent) capable of affecting a biological function of a subject or recipient to which the composition is administered. The medicament may include or may be a pharmaceutical substance or composition, such as a drug (a bioactive agent), which is given to the subject to alter a physiological condition of the subject. Accordingly, the medicament may be configured to promote healing from an injury or ailment, or the medicament may have a preventive or health-promoting activity. In some embodiments, the medicament may be a placebo that substantially lacks a bioactive agent used in a treatment plan, but which is used in the treatment plan to provide at least a psychological benefit. The medicament may be a fluid. A fluid, as used herein, includes any material that flows, such as a liquid, a gas, and/or a suspension of solid particles in a liquid or gas.

The medicament may include any suitable combination of bioactive agent(s) and excipient(s). An "excipient," as used herein, may be any component(s) of the medicament other than the bioactive agent(s). Accordingly, the excipient may have an auxiliary function different from a primary therapeutic/diagnostic function of the bioactive agent.

One or more excipients may impart fluid properties to the medicament and/or modify such fluid properties. For example, the medicament may include a fluid carrier, such as water and/or an organic fluid (such as ethanol), among others. The fluid carrier may be inert. The fluid carrier may function to dissolve (as a solvent), dilute (as a diluent), suspend, and/or propel the bioactive agent(s) and/or other excipients of the medicament. In some embodiments, the bioactive agent(s) itself may be a fluid, so that no additional fluid carrier may be needed.

The medicament may include one or more excipients that alter the viscosity, surface tension, and/or adhesiveness of the medicament. For example, the medicament may include a retention agent, such as a protein or a synthetic polymer, which may cause the medicament to remain substantially localized to a mucosal region that the medicament contacts (such as the oral or nasal mucosa). Accordingly, the retention agent may promote local absorption at the site of deposition relative to movement of the medicament to other sites after deposition, such as distal gastrointestinal regions by swallowing the deposited medicament.

One or more excipients may act as a penetration enhancer (or inhibitor). The penetration enhancer may be any agent that increases (or decreases) absorption of the bioactive agent of the medicament through a surface of the body, that is, a mucosal region, or the skin. Exemplary penetration enhancers may include alcohols such as ethanol, isopropyl alcohol, etc.; alky methyl sulfoxides, such as dimethyl sulfoxide, decylmethyl sulfoxide, and/or tetradecylmethyl sulfoxide; pyrrolidones, such as 2-pyrrolidone, N-methyl-2-pyrrolidone, and/or N-(2-hydroxyethyl)pyrrolidone); laurocapram; and various solvents, such as acetone, diemethylacetamide, dimethyl formamide, and/or tetrahydrofurfuryl alcohol. Other penetration enhancers may include amphiphiles, such as L-amino acids, anionic surfactants, cationic surfactants, amphoteric surfactants, nonionic surfactants, fatty acids, and/or fatty esters.

One or more excipients of the medicament may perform any other suitable function. Such excipients may include stabilizers, antimicrobial agents, colorants, salts, buffers, flavoring agents (imparting taste and/or smell), emulsifiers, and/or anesthetics. Further aspects of excipients and their use in medicaments are described in *Remington: The Science and Practice of Pharmacy,* 19^{th} Edition (1995).

The medicament may be administered for local or regional action, and/or for systemic action. Medicaments administered for local/regional action may act near their deposition sites. Such medicaments may include bioactive compositions having biological activity without absorption through a mucosal region, such as antibiotics, or may act at or below the mucosa. Such medicaments may be configured to treat localized infections (such as throat, sinus, or lung infections), to reduce inflammation (such as asthma), to treat cell abnormalities (such as cancer, genetic defects, etc.), and/or the like. Medicaments may be administered also or alternatively for systemic action, that is, for absorption into the bloodstream of a person, and action at one or more sites spaced from the site of absorption. Such systemic medicaments may include analgesics, antibiotics, anticancer agents, anesthetics, antidepressants, antiseizure agents, antipsychotics, CNS stimulants, drugs for treating disease, etc. Other types of medicaments that may be suitable are described in *Remington: The Science and Practice of Pharmacy,* 19^{th} Edition (1995).

The medicament may include an addictive substance or substance analog as the bioactive agent. The addictive substance may include any substance that promotes its continued intake by a person. An addictive substance may promote a strong physical and/or psychological need for intake of the substance, so that the person may be unable to terminate intake without intervention treatment. Exemplary addictive substances may include nicotine, cocaine, heroin (or other morphine derivatives/analogs), amphetamines (such as methamphetamines), alcohol, benzodiazepines, barbiturates, phencyclidine (PCP), and caffeine, among others. Addictive substance analogs may be structural and/or functional analogs of their corresponding addictive substances.

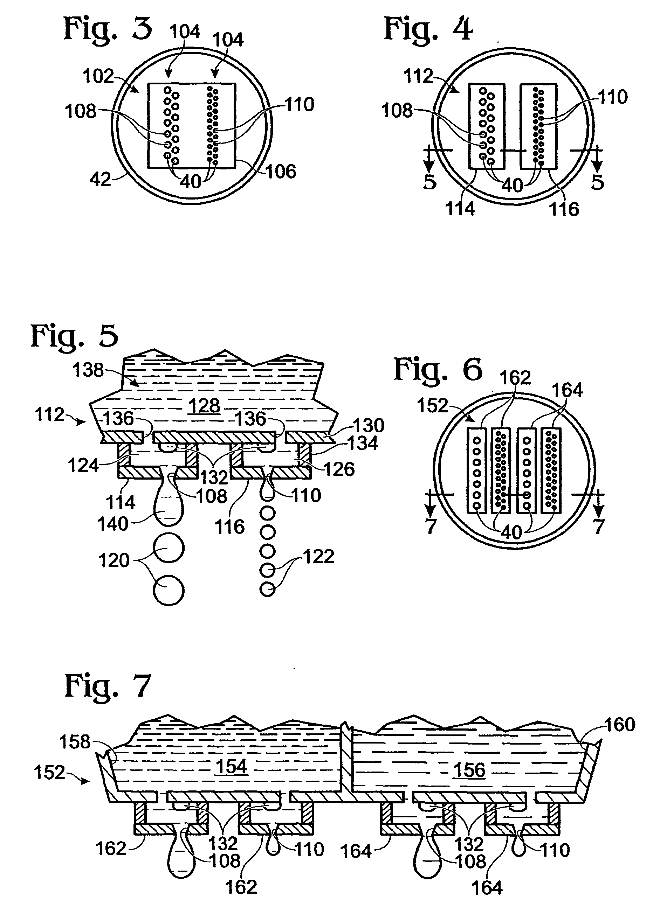

Fig. 3 shows a view an embodiment of an ejection mechanism 102 that may be included in medicament ejector 20. Ejection mechanism 102 may include a nozzle array 104 with orifices 40 of two or more different sizes included in a corresponding two or more ejection devices to enable creation of different droplet sizes of the medicament. In the present illustration, orifice plate 106 defines larger orifices 108 of larger diameter, to create larger droplets, and smaller orifices 110 of smaller diameter, to create smaller droplets. The larger and smaller orifices, provided by two ejection devices, may be spaced from one another to define spaced sub-arrays, or may be interspersed within the ejection mechanism. Interspersed orifices may form a vapor cloud of droplets having different functions. For example, droplets of a first size or composition may enable proper deposition of droplets of a second size or composition. Accordingly, the droplet of the first size/composition may function to space droplets of the second size/composition, to increase humidity, to minimize evaporation, and/or to reduce or increase droplet coalescence, among others. Ejection mechanism 102 may have any suitable number of orifices of each size (and thus any suitable number of ejection devices defined by sets of orifices), and may have any suitable number of different sizes. The orifices may be arranged in a single column or a plurality of staggered columns. In alternative embodiments, the orifices may be arranged in an orthogonal distribution, a circular distribution, an irregular distribution, or any other suitable distribution.

Fig. 4 shows a view of another embodiment of an ejection mechanism 112 that may be included in medicament ejector 20. Ejection mechanism 112 may include orifices 40 of different sizes, such as orifices 108, 110, each defined by a corresponding orifice plate 114, 116 to create spatially separate ejection devices. Each orifice plate may have any suitable thickness, size, shape, and may be formed of any suitable material. In some embodiments, the ejection mechanism may include orifice plates having different thickness, sizes, shapes, and/or formed of different materials. In some embodiments, each orifice plate may create medicament droplets of different size for different target deposition sites. The orifice plates of the ejection mechanism may be substantially parallel, for example, to fire droplets along parallel paths defined by substantially parallel orifices. Alternatively, the orifice plates and/or their orifices may be oriented obliquely relative to each other, for example, to fire droplets along nonparallel or oblique paths. Such obliquely oriented orifice plates/orifices may facilitate dispensing droplets for different target deposition sites. For example, a set of larger orifices may be oriented for ejection toward an oral mucosa, such as under the tongue or a buccal region inside the cheeks, and a set of smaller orifices may be oriented for ejection toward the back of the throat.

Fig. 5 is a fragmentary sectional view of selected portions of ejection mechanism 112 during ejection of medicament droplets 120, 122 having different sizes (that is, different diameters). Droplets 120, 122 may be ejected substantially concurrently, as shown in the present illustration, may be ejected sequentially or with partial temporal overlap within a single dose, and/or may be ejected sequentially with each droplet size restricted to a different dose.

The ejection mechanism may define firing chambers 124, 126 from which medicament 128 is ejected as droplets 120, 122. The firing chambers may be formed by a substrate 130, a firing mechanism 132, a fluid barrier 134, and orifice plate 114 or 116.

Substrate 130 may define one or more channels 136 to provide fluid communication between a fluid reservoir 138 carrying medicament 128 and chambers 124, 126. The substrate may include and/or support thin-film electronic devices and circuitry. Accordingly, the substrate may be an insulator, such as glass, or a semiconductor, such as silicon or gallium arsenide, among others.

Firing mechanism 132 may be any device that can be selectively activated to create fluid droplets ejected through orifices 108, 110 from chambers 124, 126. For example, the firing mechanism may be a thin-film device, such as a thin-film heater resistor or a piezo element included in the circuitry disposed in or adjacent the substrate. Alternatively, the firing mechanism may be defined by orifice plate 114 or 116, for example, by configuring the orifice plate to vibrate at a frequency that promotes ejection of droplets. Exemplary frequencies for ejection of droplets are about 10-100 kHz.

Fluid barrier 134 may be any layer configured to prevent lateral exit of medicament 128 from chambers 124, 126. Accordingly, the fluid barrier may be formed from substrate 130 or may be a layer of added material, such as patterned polyimide, which is connected to the substrate.

Fluid reservoir 138 may have any suitable fluid connection to firing chambers 124, 126. For example, the fluid reservoir may be disposed adjacent the substrate, as shown in the present illustration, or may be spaced from the substrate, and connected by tubing or other conduits. The fluid reservoir may be fixed, or may be a removable cartridge carrying medicament 128 (or components thereof).

Medicament 128 may be formed at any suitable time relative to ejection. The medicament may be pre-mixed, that is, having a composition created before introduction into the medicament ejector. Alternatively, the medicament may have an ejected composition created by mixing within the firing chamber, or within a mixing compartment (or compartments) in fluid communication with the firing chamber. For example, the medicament may have a concentration of a bioactive agent determined by mixing selected ratios of excipient(s) and the bioactive agent within the ejector.

Figs. 6 and 7 show views of yet another embodiment of an ejection mechanism 152 that may be included in medicament ejector 20. Ejection mechanism 152 may be configured to dispense medicaments 154, 156 held by different medicament reservoirs 158, 160. Any suitable number of medicament reservoirs may be included. Orifices of orifice plates 162 may be in fluid communication with first reservoir 158, and orifices of orifice plates 164 may be in fluid communication with second reservoir 160. In the present illustration, each medicament reservoir feeds a plurality of orifice plates. However, in alternative embodiments, each medicament reservoir may feed only one orifice plate. In addition, different medicament reservoirs may feed the same or different orifice plates. Furthermore, each medicament reservoir may feed a different size of orifice, so that medicament from each reservoir is deposited adjacent (or on) a different mucosal region. Deposition adjacent a selected deposition site or mucosal region means that the medicament droplets are deposited in the general area of the selected deposition site, but may spread somewhat before and after deposition.

The medicament reservoirs may hold the same medicament or different medicaments. Different medicaments differ in composition. Accordingly, the different medicaments may include different bioactive agents, different concentrations of a bioactive agent(s), and/or different compositions that include the bioactive agents. For example, the different medicaments may include or lack a bioactive agent, to deliver a therapeutic dose or a placebo dose, respectively. Alternatively, or in addition, the different medicaments may differ in kind, presence/absence, and/or concentration of any excipient(s). In some embodiments different medicaments may be configured to be deposited adjacent different mucosal regions, as described in more detail below. Accordingly, the different medicaments may be configured for retention and/or absorption adjacent their deposition sites. For example, a medicament may be configured to be retained adjacent an oral mucosa, to promote absorption through the oral mucosa and to reduce travel of the medicament to downstream gastrointestinal regions.

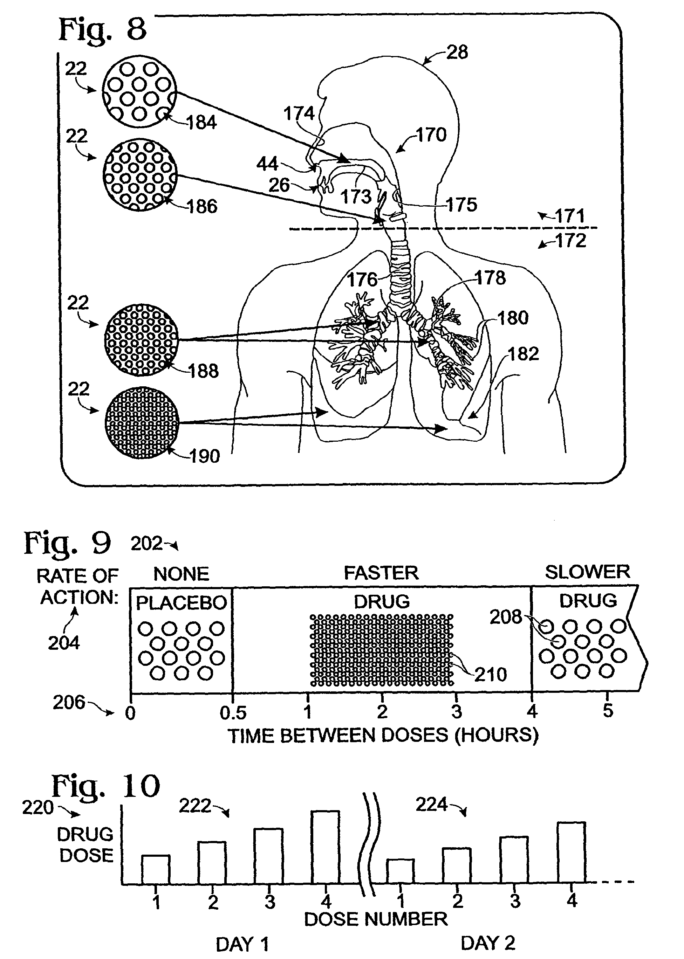

Fig. 8 schematically shows a relationship between size and deposition site for particles or fluid droplets 22 dispensed into a respiratory system 170 of person 28 by a medicament ejector. A "deposition site," as used herein, may be any landing surface onto which particles or fluid droplets of a medicament are positioned after the particles or droplets are dispensed from the ejector. Since deposition may be a statistical phenomenon, the deposition site may be a site at which a greatest percentage of the particles/droplets are placed. The deposition site may be an absorption site, that is, a site in the person at which a bioactive agent of the medicament is absorbed through a mucosa (or the skin) of the person. The deposition site may substantially define the absorption site or may be spaced from the absorption site, for example, when a medicament is deposited on the oral mucosa to be swallowed for absorption through the stomach or intestinal mucosa.

Medicament droplets 22 may be ejected from the medicament ejector into mouth 26 or nose 44, among others. Based on the size of the droplets, the droplets may be deposited relatively close to the ejector in respiratory system 170, or may travel farther into the respiratory system. In particular, larger droplets may be deposited adjacent surfaces of the upper mucosal region 171, and smaller droplets may be deposited adjacent surfaces of the lower mucosal region 172 of the respiratory system. The upper mucosal region is defined by mucous membranes of the mouth (oral mucosal region 173), the nose (nasal mucosal region 174), and the pharynx (pharyngeal mucosal region 175). The lower mucosal region is defined by larynx mucosal region 176, and pulmonary mucosae of the bronchi, bronchioles, and alveoli, that is, bronchial mucosal region 178, bronchiolar mucosal region 180, and alveolar mucosal region 182, respectively.

Particular deposition sites in the respiratory system may be determined by droplet sizes. For example, droplets having a diameter of greater than about 50 microns may provide a surface spray 184, for deposition adjacent oral mucosal region 173 or nasal mucosal region 174. Droplets having an average diameter of greater than about 10 microns or of about 10-50 microns may provide a space spray 186, for deposition adjacent upper mucosal region 171, generally by impaction. Droplets having an average diameter of less than about 10 microns may provide an aerosol spray. Droplets of about 2-10 microns may provide a sedimentation spray 188, deposited adjacent lower mucosal region 172, particularly tracheal mucosal region 176, bronchial mucosal region 178, and bronchiolar mucosal region 180, generally by sedimentation. Droplets having a diameter of about one micron, or of about 0.5 to 2 microns, may provide a diffusion spray 190 for delivering medicament to alveolar mucosal region 182 of lower mucosal region 172, generally by diffusion.

Droplets configured for particular deposition sites and selected rates of action may be outside of these size ranges when created. For example, ejected droplets may shrink or grow after ejection, during flight, based on humidity, temperature, velocity, proximity to other droplets, and composition (such as excipients in the droplets), among others. Accordingly, ejected droplet sizes may need to be adjusted to take these factors into account. Furthermore, droplets of similar initial sizes may be deposited at different sites in some cases, if the droplets have different compositions that promote different size changes while the droplets are in flight and/or if the droplets are ejected with different velocities or in different directions or regions.

Different mucosal regions may absorb medicaments at different rates, thereby creating different rates of action for the medicaments after the medicaments are dispensed. For example, the thickness of the mucosae may at least partially determine the rate of absorption, with a thinner mucosa providing faster absorption. As a general guideline, the thickness of the mucosae may decrease with increasing depth in respiratory system 170 (that is, based on the distance in the respiratory system from the mouth or nose). Accordingly, alveolar mucosal region 182 (and/or other lower mucosal regions) may absorb medicament more rapidly than oral mucosal region 173 (and/or other upper mucosal regions).

Fig. 9 is a schematic view of aspects of a treatment plan 202 that may be included in ejector 20. Treatment plan 202 may define programmatic selection of a droplet size(s) of dispensed medicament, based on a prescribed or desired rate of medicament action. The rate of action, shown at 204, may be related to a temporal schedule, such as the time elapsed since the last dose of drug, as shown at 206. Alternatively, or in addition, the treatment plan may define selection of the rate of action according to any other aspects of the user's dose record, and/or the rate of action to be selected may be specified independent of the user's dose record.

The treatment plan also may define different types of medicament to be selected based on aspects of the dose record, such as the time elapsed between doses. In the present illustration, the medicament may include a bioactive agent ("drug") with different rates of action ("faster" or "slower") or may substantially lack the bioactive agent ("placebo"), to have substantially no rate of action ("none").

The different rates of action may correspond to droplets of different sizes, such as larger droplets 208 and smaller droplets 210, which may produce slower and faster rates of absorption of the bioactive agent, respectively. These rates of absorption correspond to rates of action. Also, different sizes of droplets may be dispensed independently, that is, in different doses, or different droplet sizes and/or compositions of medicaments may be dispensed in the same dose. For example, in some embodiments, larger droplets, either placebo or drug, may be dispensed in each dose. This may provide a consistent dispensing experience for a recipient, such as a consistent feel, flavor, and/or taste independent of the rate of action.

Treatment plan 202 may be suitable for treatment of an addiction to an addictive substance, such as a smoker's addiction to nicotine. Accordingly, the drug dispensed by the ejector may be nicotine, and the dispensed placebo may lack nicotine, but may have, for example, a flavoring agent or other suitable placebo additive. A user requesting drug dispensation from the ejector may receive a dose of nicotine or placebo, based on the time elapsed since the last dose of nicotine. For example, if less than a minimum time has elapsed, such as thirty minutes in the present illustration, the ejector may dispense placebo. The placebo may have any suitable size of droplets, such as larger droplets delivered to the oral mucosal region. If greater than a minimum time has elapsed, the ejector may dispense nicotine. If larger droplets 208 of drug have not been dispensed for a suitable period of time, such as at least four hours in the present illustration, the ejector may dispense the larger droplets that provide slower action. For example, the larger droplets may be configured to be deposited adjacent the upper mucosal region, such as the oral or nasal mucosal region. The larger droplets may be suitable to achieve an elevated baseline concentration of nicotine in the user's blood. Smaller droplets 210 of nicotine also may be dispensed with larger droplets 208 in the same dose, in some embodiments. If larger droplets 208 have been dispensed within four hours, only smaller droplets 210 may be dispensed, to provide a more rapid and short-lived increase in nicotine levels in the blood. These smaller droplets may be configured to be delivered to the lower mucosal region, such as the alveolar mucosal region. Such delivery may mimic the rapid increase in nicotine provided, for example, by smoking a cigarette.

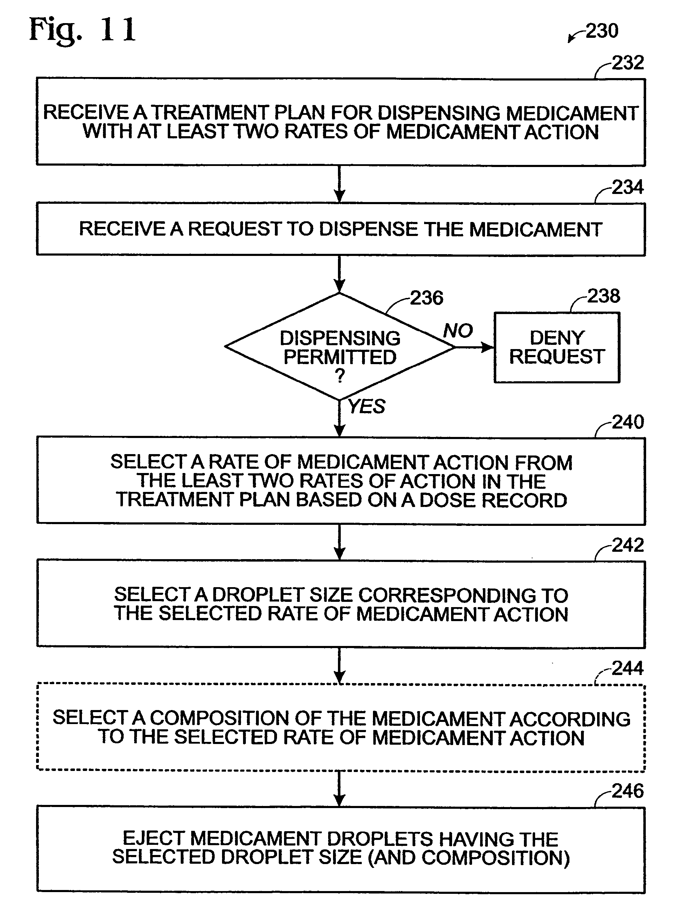

Fig. 10 shows a graph 220 in which faster-acting drug doses are plotted relative to dose number for treatment plan 202, over two consecutive days. The ejector may be configured to increase the drug dose within the day, but then decrease the drug dose over a longer period of time, such as during succeeding days. For example, to treat addiction to nicotine, increasing doses of nicotine may be administered within one day, shown at 222. This increase in nicotine dosage may be suitable, for example, to reduce craving for a cigarette. However, treatment plan 202 may be configured to wean the user from nicotine, by decreasing the total dosage over time. For example, increasing doses of nicotine may be administered on a subsequent day, but each dose may be less than the corresponding dose on the previous day, shown at 224. In other embodiments, drug dosages may be changed according to any suitable time scale, such as minutes, hours, weeks, or months. Furthermore, dosages may be decreased over time to provide a substantially constant concentration of a drug over time in a patient. For example, a first dose may "load" the patient with an effective concentration of the drug and then subsequent doses may maintain the drug near the effective concentration by replacing the drug as it is cleared from the patient. Substantially constant drug levels in the patient may be desirable for various drugs, such as antihypertensives, pain medications, and cancer treatments.

Fig. 11 is a flowchart of a method 230 of programmatically dispensing droplets of medicament to a recipient. Method 230 may be used to achieve different rates of action of the medicament, as defined by a treatment plan.

Method 230 may include receiving a treatment plan for dispensing medicament with at least two rates of medicament action, shown at 232. The treatment plan may be received by a medicament ejector through any suitable mechanism. For example, the treatment plan may be received from the recipient, may be input by a doctor, may be received along with the medicament (such as from a pharmacy), and/or may be placed in the ejector by a manufacturer of the ejector, among others.

Method 230 may include receiving a request to dispense the medicament, shown at 234. The request may be input, for example, by the recipient, or may be determined automatically by the ejector. When determined automatically, the recipient may be notified to prepare by medicament ejection.

Method 230 may include ascertaining whether dispensing the medicament is permitted, shown at 236. For example, the treatment plan and a dose record may be consulted to determine if another medicament dose, particularly with a bioactive agent, is permitted for the recipient. If not, the request may be denied, shown at 238. Denial of the request may include informing the recipient of the denial, and/or may include dispensing placebo, among others.

Method 230 may include selecting a rate of medicament action from the at least two rates of action in the treatment plan based on a dose record, shown at 240. The selected rate of action may be a single rate of action or a selected combination of action rates.

Method 230 may include selecting a droplet size and/or other medicament characteristic corresponding to the selected rate of medicament action, shown at 242. Selecting a droplet size (or sizes) may be performed at any suitable time relative to selecting rates of medicament action. For example, a relationship between droplet size and action rate may be defined within the treatment plan, so that selecting a droplet size defines an action rate and vice versa.

Method 230, optionally, may include selecting a composition of the medicament according to the selected rate of medicament action, shown at 244. Different selected rates of medicament action may be achieved with, or facilitated by, different medicament compositions. For example, larger droplets for deposition adjacent the oral mucosal region may employ a more viscous or sticky composition configured to adhere to this mucosal region. Furthermore, smaller droplets for deposition adjacent the alveolar mucosal region may employ a less viscous composition to promote formation of such smaller droplets. Alternatively, or in addition, different kinds or amounts of bioactive agents may be used in different medicaments according to a desired rate of action of the treatment plan.

Method 230 may include ejecting medicament droplets having the selected droplet size (or sizes) and/or the selected composition(s), shown at 246. The medicament droplets may be ejected into a mucosal tract of a recipient, for example, into the nose or mouth of the recipient, for deposition adjacent (generally onto) the mucosal surfaces of the respiratory system. Ejection may define the amount of medicament delivered in a dose, based, for example, on the number of droplets ejected.

In some embodiments, some or all operations of method 230 may be repeated, to dispense a different size of droplets. Each size of droplet may be dispensed according to a different temporal schedule, so that the medicament acts according to a selected combination of different action rates. Furthermore, each different size of droplets may be configured to be deposited on the upper or lower mucosal region of the respiratory system.

It is believed that the disclosure set forth above encompasses multiple distinct embodiments of the invention. While each of these embodiments has been disclosed in specific form, the specific embodiments thereof as disclosed and illustrated herein are not to be considered in a limiting sense as numerous variations are possible. The subject matter of this disclosure thus includes all novel and non-obvious combinations and subcombinations of the various elements, features, functions and/or properties disclosed herein. Similarly, where the claims recite "a" or "a first" element or the equivalent thereof, such claims should be understood to include incorporation of one or more such elements, neither requiring nor excluding two or more such elements.

## Claims

1. A method of dispensing a medicament (24), comprising: providing a treatment plan (84) having at least two rates of action (204) for a medicament (24); selecting a droplet characteristic corresponding to each of the at least two rates of action (204); and ejecting medicament droplets (22) having each droplet characteristic into a mucosal tract (171, 172) according to the treatment plan (84), thereby allowing the medicament (24) to act at two or more rates.

2. The method of claim 1, wherein selecting a droplet characteristic includes selecting a droplet size, and wherein ejecting medicament droplets (22) includes ejecting medicament droplets having each droplet size.

3. The method of claim 2, wherein selecting a droplet size includes adjusting the droplet size according to a predicted change in the droplet size produced during flight of the medicament droplets (22) after ejection.

4. The method of claim 2 or 3, wherein selecting a droplet size includes selecting a droplet size according to a deposition site for the droplet size in the respiratory system (170) of a person, the deposition site defining an absorption rate for the medicament (24) that corresponds to one of the at least two rates of action (204).

5. The method of any of claims 2-4, wherein ejecting medicament droplets (22) having each droplet size includes independently forming medicament droplets (22) of each selected size adjacent different orifices (40) of a single medicament ejection apparatus (20).

6. The method of any of claims 2-5, wherein ejecting medicament droplets having each droplet size includes ejecting at least a subset of the medicament droplets (22) of each droplet size at different times (206).

7. The method of any of claims 2-6, wherein ejecting medicament droplets (22) having each droplet size includes ejecting at least a subset of the medicament droplets of each droplet size within a single dose.

8. The method of any of claims 2-7, which further comprises selecting a composition for each droplet size, the composition being selected from a set of compositions having different amounts of a bioactive agent.

9. The method of any of claims 1-8, wherein providing a treatment plan (84) includes providing a treatment plan (84) to treat addiction to nicotine, and wherein the medicament (24) includes nicotine or a nicotine analog.

10. The method of any of claims 2-9, wherein selecting a droplet size includes selecting a different size of medicament droplet (22) for each rate of action (204).

11. A method of dispensing a medicament (24), comprising: providing an ejector (20) configured to selectively eject medicament droplets (22) of at least two sizes, a first size being configured for deposition adjacent an oral or nasal mucosa (171) and a second size being configured for deposition adjacent a pulmonary mucosa (172); and ejecting the medicament droplets (22) of each size according to a different temporal schedule into a mucosal tract from the ejector (20).

12. The method of claim 11, which further comprises selecting a treatment program (84) for addiction to a substance, the treatment program defining the different temporal schedule for ejecting the medicament droplets (22) of each size, wherein ejecting the medicament droplets (22) includes ejecting droplets of the substance or an analog thereof according to the different temporal schedule.

13. An apparatus (20) for dispensing medicaments (24), comprising: a plurality of reservoirs (158, 160), each reservoir holding a different medicament composition; and an ejection mechanism (102) in fluid communication with the reservoirs (158, 160) and including a plurality of ejection devices, each ejection device being configured to independently dispense at least one medicament composition held by least one of the reservoirs (158, 160).

14. The apparatus of claim 13, wherein each reservoir holds at least one of a different concentration of a drug and a different drug.

15. The apparatus of claim 13 or 14, wherein each ejection device includes a set of orifices (40) from which the at least one medicament composition is dispensed as droplets (22), and wherein the orifices (40) have a different size when compared among the ejection devices.

16. A medicament ejector (20), comprising: a plurality of reservoirs (158, 160) holding an excipient and a drug; and an ejection mechanism (102) in fluid communication with the reservoirs and including at least two ejection devices, each ejection device being configured to independently dispense a different medicament composition that includes at least one of the excipient and the drug.

17. The medicament ejector (20) of claim 16, wherein the excipient and the drug are in different reservoirs (158, 160), the medicament ejector (20) further comprising a mixing chamber configured to mix the excipient and the drug before ejection by the ejection mechanism (102).

18. The medicament ejector (20) of claim 16, wherein the excipient and the drug are in different reservoirs (158, 160), one of the ejection devices being configured to eject the drug, and another of the ejection devices being configured to eject the excipient for in-flight dilution of the drug.

19. A medicament ejector (20), comprising: an ejection mechanism (102) including a plurality of ejection devices, at least one of the devices being configured to provide a space spray (186) of a medicament (24) and at least another of the devices being configured to provide an aerosol spray (188, 190) of the medicament; and a controller (50) coupled to the ejection mechanism (102) and configured to signal the ejection mechanism (102) to provide each corresponding spray.

20. The medicament ejector of claim 19, wherein the space spray (186) includes a first set of droplets (22) having an average diameter of greater than about 10 microns, and wherein the aerosol spray (188, 190) includes a second set of droplets (22) having an average diameter of less than about 10 microns.
